# EUROPÄISCHE PATENTSCHRIFT

(11) **EP 1 735 033 B1**
(45) Veröffentlichungstag und Bekanntmachung des Hinweises auf die Patenterteilung: **05.01.2011**
(21) Anmeldenummer: 04761874.9
(22) Anmeldetag: 20.08.2004
(51) Int. Cl.: A61M 5/50

(54) **KOLBENKOPF FÜR EINE ZWEITEILIGE INJEKTIONSSPRITZE**
PLUNGER HEAD FOR A TWO-PIECE INJECTION SYRINGE
TETE DE PISTON POUR SERINGUE D'INJECTION EN DEUX PARTIES

(30) Priorität: 16.04.2004 CH 671042004
(43) Veröffentlichungstag der Anmeldung: 27.12.2006
(73) Patentinhaber: Schöttli, Theodor, 8253 Diessenhofen (CH)
(72) Erfinder: Schöttli, Theodor, 8253 Diessenhofen (CH)
(74) Vertreter: Gachnang, Hans Rudolf
(86) Internationale Anmeldenummer: PCT/CH2004/000530
(87) Internationale Veröffentlichungsnummer: WO 2005/099795

(56) Entgegenhaltungen:
- WO-A-00/40284
- WO-A-00/41494
- WO-A-2004/078243
- US-A1- 2003 212 367
- US-B1- 6 217 550

## Beschreibung

Gegenstand der Erfindung ist ein Kolbenkopf für eine zweiteilige Injektionsspritze gemäss Oberbegriff des Patentanspruchs 1.

Injektionsspritzen sind in vielen Ausführungen bekannt. Sie umfassen einen Spritzenzylinder mit einer Kupplungsvorrichtung für eine Injektionsnadel und einen Kolben mit einem Kolbenkopf und einem Kolbenschaft sowie einen Fingerflansch zum Betätigen des Kolbens.
Solche Injektionsspritzen werden nicht ausschliesslich durch ausgebildetes Personal verwendet, sondern dienen vermehrt zur Injektion von Betäubungsmitteln und gelangen dadurch in die Hände von nicht ausgebildeten Personen, welche bezüglich Sterilität keine Sorgfalt walten lassen.

Es sind aus diesem Grunde bereits Injektionsspritzen bekannt geworden, mit denen der Erstgebrauch angezeigt werden soll bzw. die nur ein einziges Mal benutzbar sein sollen. Die Möglichkeit, die Spritze nur ein einziges Mal zu benutzen, schützt nebst Rauschmittelsüchtigen auch andere Kranke vor allem in unterentwickelten Ländern davor, in Kontakt mit verseuchtem Blut an einer bereits benutzten Spritze zu gelangen.

Aus der WO 00/40284 ist eine Injektionsspritze mit einem Spritzenzylinder bekannt, bei der der Kolbenkopf mit fadenförmigen Stegen direkt am Schaft des Kolbens angebunden ist. Bricht einer der fadenförmigen Stege, so kann sich der Kolbenkopf innerhalb des Zylinders schrägstellen und es besteht dabei die Gefahr, dass ein Teil des Injektionsmittels am schräggestellten Kolbenkopf vorbei hinter letzteren fliessen kann. Zudem lässt sich die Injektionsflüssigkeit bei schräggestelltem Kolbenkopf nicht mehr vollständig injizieren.

Aus der US 2003/0212367 ist weiter eine Wegwerfspritze bekannt, bei welcher der Kolbenkopf ringförmig auf einer Verlängerung des Kolbenschafts aufgesteckt ist und bei der der den Kolbenkopf überragende Teil der Verlängerung am Ende des Injektionsvorgangs sich in die Nadelaufnahme hineinschiebt und diese beim Zurückziehen des Schafts und dem Kolben zusammen mit der Nadel in den Zylinder hineinzieht. Bei dieser Spritze besteht nie die Gefahr, dass der Kolbenkopf vor oder während des Injizierens sich schrägstellen bzw. kippen kann. Um die Gesamtlänge der Spritze nach dem Zurückziehen der Nadel in den Zylinder zu verkürzen, kann der Schaft hinter dem Kolbenkopf abgebrochen werden. Ein Wulst im Zylinder verhindert das Herausziehen des Kolbenkopfs und der Nadel. Das erneute Einziehen von Injektionsflüssigkeit in die Spritze ist in keinem Fall gewährleistet, wenn die Injektion kurz vor dem Ende unterbrochen wird und so keine Verbindung zwischen dem Schaft des Kolbens und der Nadelaufnahme entstehen kann.

Aus der US 6,217,550 ist weiter eine Injektionsspritze bekannt, bei der der Kolbenkopf scheibenförmig ausgebildet ist und über Sollbruch-Stellen mit dem Schaft verbunden ist. Bricht einer die Sollbruch-Stelle bildenden Stege, so stellt sich der Kolbenkopf schräg oder kippt und es kann entweder der Injektionsvorgang nicht vollständig durchgeführt werden oder es tritt Injektionsflüssigkeit in den rückwärtigen Teil der Spritze und kann entlang dem Schaft nach aussen austreten.

Die Aufgabe der vorliegenden Erfindung besteht nun darin, eine Injektionsspritze derart weiterzubilden, dass nach einmaligem Gebrauch ein erneutes Einziehen von Flüssigkeit unmöglich ist.

Gelöst wird diese Aufgabe durch eine Injektionsspritze mit den Merkmalen des Patentanspruchs 1. Vorteilhafte Ausgestaltungen der Erfindung sind in den abhängigen Ansprüchen definiert.

Der nach der Erfindung ausgebildete Kolbenkopf bricht am Ende des Injektionsvorgangs oder spätestens beim Zurückziehen vom Schaft ab und lässt sich damit nicht mehr zurückziehen. Er bleibt im Spritzenzylinder stecken. Durch die Anordnung einer parallel zum Kolbenkopf angeordneten und in einem axialen Abstand liegenden Führungsscheibe wird sichergestellt, dass der Kolbenkopf nach dem Brechen eines einzigen Steges sich nicht neigen und dadurch wegen seines kleineren projizierenden Querschnitts wieder zurückgezogen oder zurückgedrückt werden kann.

In der Ausgestaltung der erfindungsgemässen Spritze mit Stegen kann die Bruchlast in Abhängigkeit der Verschieberichtung des Spritzenkolbens unterschiedlich gross gestaltet werden. Dies ermöglicht das Aufziehen der Flüssigkeit, ohne Gefahr zu laufen, dass die Stege brechen. Bei der nachfolgenden Injektion hingegen brechen die Stege bereits bei wenigen beispielsweise 7-10 Newton Schubkraft. Dies beeinträchtigt die Funktion der Spritze nicht, weil der Spritzeninhalt dennoch ausgestossen werden kann. Das Schaftende schiebt den Kolbenkopf in jedem Fall nach vorne. Das Schaftende liegt dann an der das Ende der Bohrung überspannenden Dichtungskappe an.

Die Herstellung der Spritze, d.h. sowohl des Zylinders und des Kolbens ist kostengünstig, da nur zwei Teile gefertigt und ein einziger Teil montiert werden müssen.

Anhand illustrierter Ausführungsbeispiele wird die Erfindung näher erläutert. Es zeigen
- Figur 1: eine perspektivische Darstellung einer Einwegspritze mit teilweise eingeschobenem Kolbenkopf,
- Figur 2: einen Längsschnitt durch eine Spritze mit in Ausgangsstellung befindlichem Kolbenkopf,
- Figur 3: einen Längsschnitt durch die Spritze mit vollständig vorgeschobenem Kolbenkopf, der vom Schaft abgetrennt ist,
- Figur 4: einen Querschnitt durch den Kolbenkopf und das Schaftende längs Linie IV -IV in Fig. 5,
- Figur 5: einen Längsschnitt durch den Kolbenkopf und das Schaftende,
- Figur 6: einen Längsschnitt durch den Kolbenkopf in einer weiteren Ausgestaltung der Erfindung,
- Figur 7: einen Längsschnitt durch den Kolbenkopf in einer weiteren Ausgestaltung der Erfindung,
- Figur 8: einen Längsschnitt durch eine Spritze mit in Ausgangsstellung befindlichem Kolbenkopf ohne zusätzliche Dichtungskappe,
- Figur 9: eine Ansicht eines Kolbenkopfs einer weiteren Ausführungsform der Erfindung,
- Figur 10: eine Ansicht eines Kolbenkopfs mit einer Führungsscheibe.

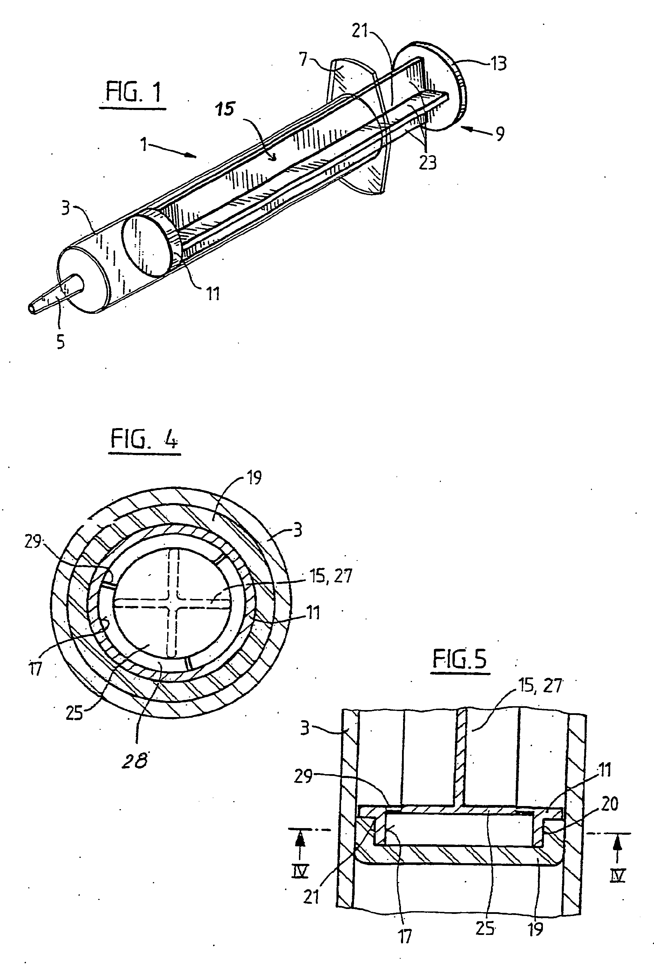

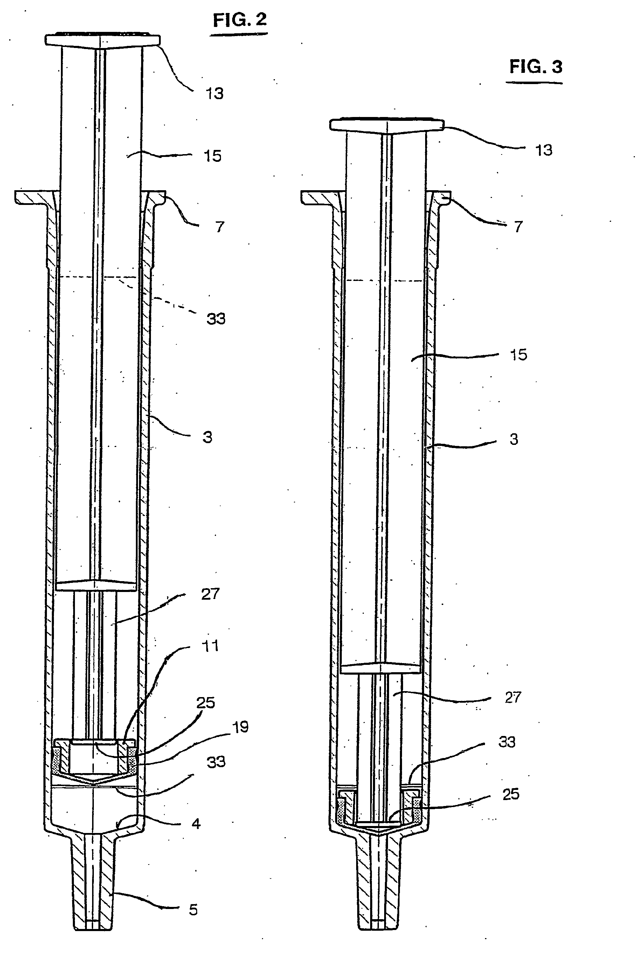

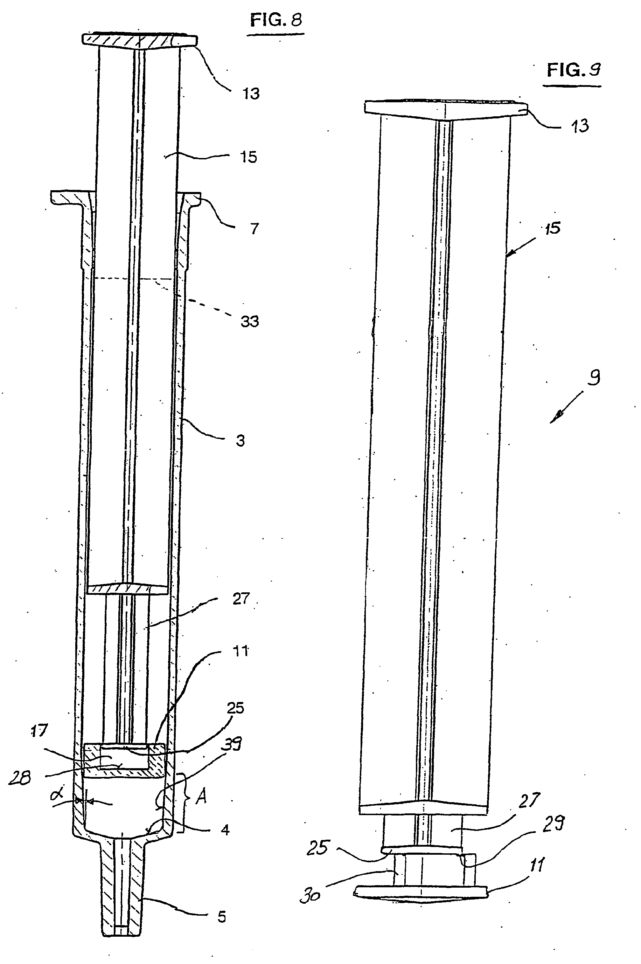

In der perspektivischen Übersichtsdarstellung gemäss Figur 1, ist mit Bezugszeichen 1 eine Einwegspritze bezeichnet. Diese umfasst einen Spritzenzylinder 3 mit einer Kupplungsvorrichtung für eine Injektionsnadel (nicht aufgesetzt), z.B. einen Nadelkonus 5, sowie zwei radial abstehende Griffplatten 7. Im Spritzenzylinder 3 ist ein teilweise vorgeschobener Spritzenkolben 9 dargestellt, an dessen vorderem Schaftende ein Kolbenkopf 11 aufgesetzt und an dessen hinterem Schaftende ein Fingerflansch 13 ausgebildet sind. Zwischen dem Kolbenkopf 11 und dem Fingerflansch 13 liegt ein beispielsweise einen kreuzförmigen Querschnitt aufweisender Schaft 15. Die beiden Hauptteile, Spritzenzylinder 3 und Spritzenkolben 9, sind aus Kunststoff durch Spritzen hergestellt. Im Kolbenkopf 11 ist eine axial verlaufende, den Kolbenkopf 11 ganz (vgl. Figuren 2 bis 7) oder nur teilweise (Figuren 8 und 9) durchdringende Bohrung 17 eingelassen. Eine vorzugsweise kappenförmige Dichtung 19 kann in der ersten Ausführungsform den Kolbenkopf 11 peripher und seine stirnseitige Bodenfläche umgeben. Die kappenförmige Dichtung 19 erstreckt sich über mindestens einen Teil der Peripherie 20 des Dichtungskolbens 11 und liegt dort innerhalb eines umlaufenden Absatzes 21 eingebettet (vgl. Figur 5). Der kreuzförmige Schaft 15 weist mindestens im vorderen Abschnitt einen Durchmesser auf, der kleiner ist als der Durchmesser der Bohrung 17.

In einer Ausgestaltung der Erfindung sitzt auf dem Ende des Schafts 15 eine Scheibe 25, deren Durchmesser gleich oder grösser ist als der Durchmesser der Balken 23 des Schafts 15, jedoch kleiner ist als der Durchmesser der Bohrung 17.

Zwischen der Peripherie der Scheibe 25 bzw. dem vorderen, vorzugsweise eine geringere Dicke aufweisenden Schaftabschnitt 27 und der Bohrung 17 ergibt sich ein Spalt 28.

Die Verbindung zwischen dem Schaftabschnitt 27 bzw. der Scheibe 25 und dem Kolbenkopf 11 erfolgt durch feine faden-, stäbchen- oder filmförmige Stege 29, welche sich von dem Balken 23 am Schaftabschnitt 27 oder von der Peripherie der Scheibe 25 radial an die Wandung der Bohrung 17 erstrecken und mit dieser verbunden sind. Die Verbindung der im Querschnitt runden oder eckigen Stege 29 kann am Ende der Bohrung 17 oder axial versetzt im Innern der letzteren erfolgen. Die Festigkeit der Stege 29 ist derart bemessen, dass sie bei Überschreiten einer vorgebbaren Axialkraft, z.B. 10N vom Schaft 15 oder der Scheibe 25 abgeschert werden. Eine nähere Erläuterung dieses Vorgangs erfolgt bei der Funktionsbeschreibung.

Im Hubraum des Spritzenzylinders 3 kann in einem Abstand vom Spritzenzylinderboden, der grösser ist als die axiale Ausdehnung des Kolbenkopfs 11 ein umlaufender Wulst 33 ausgebildet sein, welcher in den Hubraum des Zylinders 3 und damit in den Verschiebeweg des Kolbenkopfs 11 hineinragt und dessen Querschnitt verkleinert. Die radiale Ausdehnung des Wulstes 33 ist sehr gering und hängt vom Durchmesser des Spritzenzylinders 3 ab.

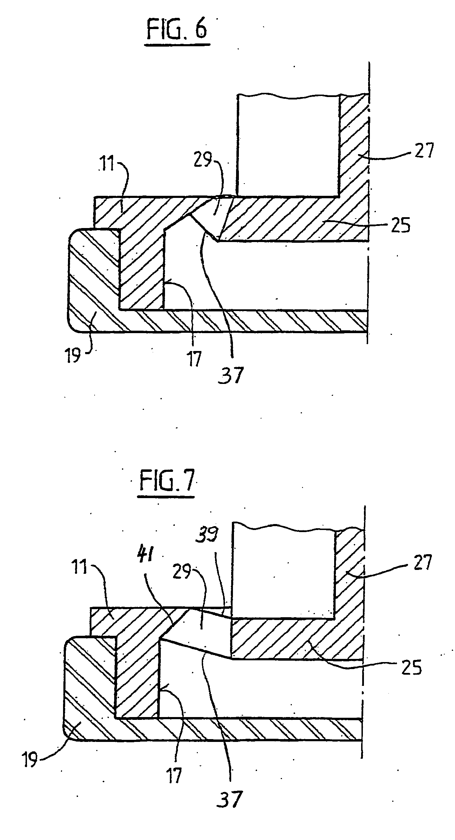

In der Ausgestaltung der Erfindung nach den Figuren 6 und 7 weisen die Stege 29 einen viereckigen Querschnitt auf, wobei die erste, dem Kolbenkopfboden zugewendete Kante 37 der Stege geneigt verläuft. Die dem Kolbenkopfboden abgewendete zweite Kante 39 kann parallel zum Kolbenkopfboden oder parallel zur ersten Kante 37 verlaufen. Die Anbindungsbereiche der Stege 29 am Kolbenkopf 11 und am Schaftabschnitt 27 oder an der auf dem Schaftabschnitt 27 sitzenden Scheibe 25 können parallel verlaufen oder - wie in den Beispielen in den Figuren 6 und 7 dargestellt - kann die Bohrung 17 im Bereich der Stege 29 einen konisch verlaufenden Abschnitt 39 aufweisen. Analog kann auch die Peripherie der Scheibe 25 konisch verlaufend ausgebildet sein, d.h. die Scheibe 25 weist die Gestalt eines Kegelstumpfs auf (Figur 6).

In der Ausgestaltung der Erfindung gemäss Figur 8 ist am Kolbenkopf 11 keine zusätzliche elastische Dichtung angebracht, sondern der Kolbenkopf 11 liegt mit seiner Peripherie direkt auf der Wandung des Spritzenzylinders 3 an. Der Schaftabschnitt 27 ist wiederum mittels feinen Stegen 29 mit dem Kolbenkopf 11 verbunden, wobei im Kolbenkopf 11 eine Sackbohrung 17 ausgebildet ist, in welche der Schaftabschnitt 27 nach dem Brechen der Stege 29 einfahren und am Boden 28 anstossen kann.

In der Ausgestaltung der Erfindung nach Figur 9 ist am Schaftende wiederum ein Kolbenkopf 11 dargestellt, der direkt mit seiner Peripherie an der Wandung des Spritzenzylinders dichtend anliegt. Die Verbindung zwischen dem Kolbenkopf 11, der hier scheibenförmig ausgebildet ist, und dem Schaftabschnitt 27 erfolgt durch beispielsweise drei Füsse 30, die einseitig fest mit dem scheibenförmigen Teil des Dichtungskopfs 11 verbunden sind und auf der anderen Seite je durch einen Steg 29 mit dem vorderen Ende des Schaftabschnitts 27 bzw. mit der Scheibe 25. Vorzugsweise entspricht die Länge der Füsse 23 dem Abstand der Scheibe 25 vom unteren Ende des oberen Teils des Schafts 15.

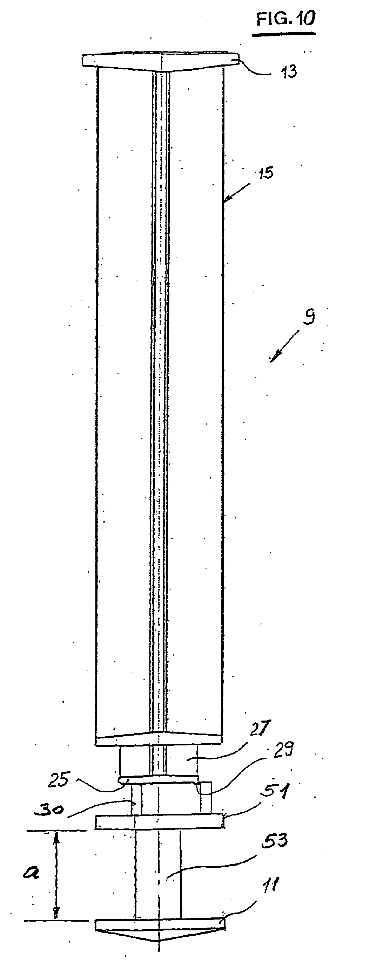

In der Ausgestaltung der Erfindung gemäss Figur 10 sind die Füsse 30 vorne nicht unmittelbar am Kolbenkopf 11 befestigt, welcher als Dichtungsscheibe fungiert, sondern an einer Führungsscheibe 51. Die Führungsscheibe 51 trägt den Kolbenkopf 11. Dieser sitzt auf einer zentral angeordneten runden oder mehreckigen Säule 53. Der axiale Abstand a zwischen dem Kolbenkopf 11 und der Führungsscheibe 51 ist derart bemessen, dass ein Kippen der beiden durch die Säule 53 verbundenen Teile auch beim Brechen nur eines einzigen Steges 29 nicht möglich ist.

Im folgenden wird die Funktionsweise der Einwegspritze 1 näher erläutert.
Beim Hersteller der Spritze 1 wird nach dem Spritzen der Einzelteile der Spritzenkolben 9 direkt in den Spritzenzylinder 3 eingeschoben. Das Einschieben erfolgt allerdings nur bis das vordere Ende des Kolbenkopfs den Anfang eines konischen Bereichs A (siehe Figur 8) erreicht hat, sofern der Spritzenzylinder 3 mit einem solchen einen Konuswinkel alpha aufweisenden Bereich A ausgerüstet ist. Bei einem durchgehend zylindrischen Spritzenzylinder 3 kann der Kolben 9 bis zum Kolbenboden 4 vorgeschoben werden.

Der Benutzer der Spritze 1 füllt diese in herkömmlicher Weise, indem er den Spritzenkolben 7 zurückzieht. Nach der Entlüftung durch Ausblasen der Luft im vorderen Teil des Spritzenzylinders 3 erfolgt die Injektion, wobei der Kolbenkopf 11 zur vollständigen Entleerung bis zum Anschlag am Boden 4 am nadelseitigen Ende des Spritzenzylinders 3 vorgeschoben wird. Auf den letzten Millimetern des Vorschubs beim überfahren des Wulstes 33, falls ein solcher vorgesehen ist, oder beim Einfahren in den konischen Bereich A und/oder beim Auspressen des letzten Rests der Flüssigkeit brechen die Stege 29 wegen des erhöhten Widerstands und der Schaft 15 gleitet in der Bohrung 17 bzw. zwischen den Füssen 30 (Fig. 9) nach vorn und stösst am Grund 28 der Bohrung 17 bzw. die Füsse 30 am Schaftende an.

Der Kolbenkopf 11 lässt sich, dank der Führungsscheibe 51 stets axial geführt, sicher bis ans Hubraumende am Boden 4 des Spritzenzylinders 3 vorschieben, obwohl die Stege 29 gebrochen sind.
Ein erneutes Aufziehen von Flüssigkeit ist ausgeschlossen. Die Einwegspritze 1 ist damit unbrauchbar geworden.

## Patentansprüche

1. Kolbenkopf (11) für eine zweiteilige Injektionsspritze (1) aus Kunststoff, mit einem Spritzenzylinder (3) mit einer am vorderen Zylinderende ausgebildeten Kupplungsvorrichtung (5) zum Aufsetzen einer Injektionsnadel, einem am hinteren Zylinderende ausgebildeten Griffplattenpaar (7), einem im Hubraum des Spritzenzylinders (3) längsführbaren Spritzenkolben (9), letzterer umfassend einen Schaft (15,27) mit dem scheibenförmig ausgebildeten Kolbenkopf (11) an dem in den Spritzenzylinder (3) eintauchenden ersten Ende und einen Fingerflansch (13) am zweiten Ende, wobei der Kolbenkopf (11), der Schaft (15,27) und der Fingerflansch (13) einstückig durch Spritzen hergestellt sind,
wobei zwischen dem scheibenförmigen Kolbenkopf (11) und dem Ende des Schafts (15) in einem axialen Abstand (a) eine Führungsscheibe (51) eingesetzt ist, welche durch eine Säule (53) derart mit dem Kolbenkopf (11) verbunden ist, dass ein Kippen des Kolbenkopfs (11) verhindert wird,
**dadurch gekennzeichnet**
- **dass** die Führungsscheibe (51) mit faden-, stäbchen- oder filmförmigen Stegen (29), die von der Führungsscheibe (51) zum Schaft (15) verlaufen, mit dem Schaft (15) verbunden ist und
- **dass** an der Führungsscheibe (51) Mittel zum Abstützen der Führungsscheibe (51) am Schaftende nach dem Brechen der Stege (29) ausgebildet sind,
und
- **dass** als Mittel zum Abstützen an der Führungsscheibe (51) axial verlaufende Stützfüsse (30) angeformt sind, an deren freien Enden die Stege (29), welche die Verbindung zum Schaft (15) herstellen, angeordnet sind.

2. Kolbenkopf (11) nach Anspruch 1, **dadurch gekennzeichnet, dass** drei Stege (29) in drei radial liegenden Ebenen von der Führungsscheibe (51) zum vorderen Ende des Schafts (27) verlaufend ausgebildet sind.

3. Kolbenkopf (11) nach einem der Ansprüche 1 oder 2, **dadurch gekennzeichnet, dass** die Stege (29) an einer am Ende des Schafts (15) ausgebildeten Scheibe (25) befestigt sind.

4. Kolbenkopf (11) nach Anspruch 3, **dadurch gekennzeichnet, dass** die Kanten (37,39) der Stege (29) sich mit zunehmendem radialen Abstand vom Schaft (15) oder der Scheibe (25) sich nähern und die Kontaktfläche mit der Führungsscheibe (51) kleiner ist als mit der Scheibe (25) oder dass die Stege (29) in einer kegelstumpfförmigen Fläche verlaufend ausgebildet sind.

5. Kolbenkopf (11) nach einem der Ansprüche 1 bis 4, **dadurch gekennzeichnet, dass** in einem Abstand zum vorderen und/oder hinteren Ende des Hubraums im Spritzenzylinder (3) je ein ganz oder teilweise umlaufender, den Querschnitt verengender Wulst (33) und/oder am vorderen Ende ein konisch sich verengender Abschnitt (A) ausgebildet ist.

## Claims

1. A piston head (11) for a two-part injection syringe (1) of plastic, with a syringe cylinder (3) with a coupling device (5) for attaching an injection needle, said coupling device being formed at the front cylinder end, with a grip plate pair (7) formed at the rear cylinder end, with a syringe piston which is longitudinally guidable in the travel space of the syringe cylinder (3), said syringe piston comprising a shank (15, 17) with the piston head (11) formed in a disc-like manner, at the first end which immerses into the syringe cylinder (3), and with a finger flange (13) at the second end, wherein the piston head (11), the shank (15, 27) and the finger flange (13) are manufactured as one piece by way of injection moulding,
wherein a guide disk (51) is inserted between the disk-like piston head (11) and the end of the shank (15), at an axial distance (a), and this guide disk is connected to the piston head (11) by way of a column (53), in a manner such that a tilting of the piston head (11) is prevented, **characterised in that**
- the guide disk (51) is connected to the shank (51) with thread-like, rod-like or film-like webs (29) which run from the guide disk (51) to the shank (15),
- that means for supporting the guide disk (51) on the shank end after the breaking of the webs (29), are formed on the guide disk (51),
and
- that axially running support feet (30) are integrally formed on the guide disk (51) as means of support, at whose free end the webs (29) which create the connection to the shank (15), are arranged.

2. A piston head (11) according to claim 1, **characterised in that** three webs (29) are designed running in three radially lying planes, from the guide disk (51) to the front end of the shank (27).

3. A piston head (11) according to one of the claims 1 or 2, **characterised in that** the webs (29) are fastened on a disk (25) formed at the end of the shank (15).

4. A piston head (11) according to claim 3, **characterised in that** the edges (37, 39) of the webs (29) approach one another with an increasing radial distance to the shank (15) or to the disk (25), and the contact surface with the guide disk (51) is smaller than with the disk (25) or that the webs (29) are designed running in a truncated-cone-like surface.

5. A piston head (11) according to one of the claims 1 to 4, **characterised in that** in each case a completely or partially peripheral bead (33) narrowing the cross section, is formed in the syringe cylinder at a distance to the front and/or rear end of the travel space, and/or a conically narrowing section (A) is formed at the front end.

## Revendications

1. Tête de piston (11) pour une piqûre d'injection à deux parties (1) en matière plastique, comprenant un cylindre de piqûre (3) muni d'un dispositif d'accouplement (5) réalisé sur une extrémité avant de cylindre pour la mise en place d'une aiguille d'injection, muni d'une paire de plaquettes d'emprise (7) réalisée sur l'extrémité arrière de cylindre, muni d'un piston de piqûre (9) qui peut être guidé en longueur dans l'espace de course du cylindre de piqûre (3), dans lequel ce dernier comporte une tige (15, 27) munie de la tête de piston (11) réalisée en forme de rondelle et sur laquelle une première extrémité qui plonge dans le cylindre de piqûre (3) ainsi qu'une bride pour les doigts (13) sur la seconde extrémité sont fabriquées en un seul tenant par moulage par injection,
**caractérisée en ce**
- **que** la rondelle de guidage (51) est reliée à la tige (15) par des nervures (29) en forme de fil, de petite tige ou de film, qui s'étendent depuis la rondelle de guidage (51) jusque vers la tige (15), et en ce
- **que**, sur la rondelle de guidage (51), des moyens pour soutenir la rondelle de guidage (51) sont réalisés sur l'extrémité de tige après la brisure des nervures (29), et en ce
- **que** des pieds de soutien (30) qui s'étendent axialement sont conformés sur la rondelle de guidage (51) comme moyens pour le soutien, sachant que les nervures (29) qui établissent la liaison vers la tige (15) sont agencées sur leurs extrémités libres
dans laquelle une rondelle de guidage (51) est mise en place entre la tête de piston en forme de rondelle (11) l'extrémité de la tige (15) à une distance axiale (a) et laquelle est reliée à la tête de piston (11) par une colonne (53) de telle sorte qu'un basculement de la tête de piston (11) est empêché.

2. Tête de piston (11) selon la revendication 1, **caractérisée en ce que** trois nervures (29) sont réalisées en s'étendant dans trois plans placés radialement depuis la rondelle de guidage (51) jusque vers l'extrémité avant de la tige (27).

3. Tête de piston (11) selon l'une ou l'autre des revendications 1 et 2, **caractérisée en ce que** les nervures (29) sont fixées sur une rondelle (25) réalisée sur l'extrémité de la tige (15).

4. Tête de piston (11) selon la revendication 3, **caractérisée en ce que** les arêtes (37, 39) des nervures (29) se rapprochent de la tige (15) ou de la rondelle (25) avec une distance radiale croissante et **en ce que** la surface de contact avec la rondelle de guidage (51) est plus petite qu'avec la rondelle (25) ou **en ce que** les nervures (29) sont réalisées en s'étendant dans une surface en forme de tronc de cône.

5. Tête de piston (11) selon l'une des revendications 1 à 4, **caractérisée en ce que**, à une distance envers l'extrémité avant et/ou arrière de l'espace de course dans le cylindre de piqûre (3), une collerette (33) qui rétrécit la section transversale et qui s'étend sur toute la surface ou sur une partie de surface et/ou un tronçon (A) qui se rétrécit à la manière d'un cône sur l'extrémité avant est réalisé à chaque fois.
